(19) Europäisches Patentamt / European Patent Office / Office européen des brevets

(11) **EP 3 454 061 B1**

(12) **FASCICULE DE BREVET EUROPEEN**

(45) Date de publication et mention
de la délivrance du brevet:
**11.03.2020 Bulletin 2020/11**

(51) Int Cl.:
***G01N 33/24*** *(2006.01)*     ***G01N 31/12*** *(2006.01)*

(21) Numéro de dépôt: **18191048.0**

(22) Date de dépôt: **28.08.2018**

(54) **PROCEDE POUR LA QUANTIFICATION DU SOUFRE PYRITIQUE ET DU SOUFRE ORGANIQUE D'UN ECHANTILLON DE ROCHE**

VERFAHREN FÜR DIE QUANTIFIZIERUNG VON PYRITSCHWEFEL UND ORGANISCHEM SCHWEFEL EINER GESTEINSPROBE

METHOD FOR QUANTIFYING THE PYRITIC SULPHUR AND ORGANIC SULPHUR OF A ROCK SAMPLE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.09.2017 FR 1758413**

(43) Date de publication de la demande:
**13.03.2019 Bulletin 2019/11**

(73) Titulaire: **IFP Energies nouvelles
92852 Rueil-Malmaison Cedex (FR)**

(72) Inventeurs:
 • **ABOUSSOU, Anabel
 92500 RUEIL-MALMAISON (FR)**
 • **LAMOUREUX-VAR, Violaine
 78400 CHATOU (FR)**
 • **PILLOT, Daniel
 78100 ST GERMAIN EN LAYE (FR)**
 • **KOWALEWSKI, Isabelle
 78870 BAILLY (FR)**

 • **DOLIGEZ, Brigitte
 75014 Paris (FR)**
 • **GARCIA, Bruno
 92200 NEUILLY SUR SEINE (FR)**
 • **WAGNER, Thomas
 EDINBURGH, SCOTLAND, EH14 4AS (GB)**
 • **BUCKMAN, James Oliver
 EDINBURG, SCOTLAND, EH14 4AS (GB)**
 • **MARZ, Christian
 LEEDS, LS12 2JB (GB)**

(74) Mandataire: **IFP Energies nouvelles
Département Propriété Industrielle
Rond Point de l'échangeur de Solaize
BP3
69360 Solaize (FR)**

(56) Documents cités:
**EP-A1- 2 342 557    US-A- 4 845 040
US-B1- 6 319 717**

EP 3 454 061 B1

## Description

La présente invention concerne le domaine technique de l'industrie pétrolière, et plus particulièrement le domaine de l'exploration et de l'exploitation d'une formation géologique dans laquelle sont piégés des hydrocarbures.

Plus précisément, la présente invention concerne la caractérisation et la quantification du soufre présent au sein d'une roche sédimentaire, telle qu'une argile marine riche en matière organique.

Afin de répondre à la demande énergétique croissante, l'industrie pétrolière se tourne de plus en plus vers la production de pétroles bruts non conventionnels, qui sont plus riches en soufre que les pétroles conventionnels. Or la teneur en soufre d'un pétrole brut non conventionnel, ainsi que le type de composés organiques soufrés qu'il contient, sont des paramètres clés de la qualité de ce pétrole et des produits de raffinage qui en sont issus. De plus, les règlementations imposent des teneurs en soufre de plus en plus faibles pour les produits issus du raffinage. Pour ces raisons, il est important de savoir caractériser et quantifier précisément le soufre présent dans les roches qui sont à l'origine de ces pétroles bruts soufrés.

Dans le cas de roches mères pétrolières, les deux principaux composés soufrés sont le soufre organique et le soufre pyritique. La quantification du soufre organique, indépendamment du soufre pyritique, est d'une grande importance en exploration pétrolière, car elle permet de connaître exactement la quantité de soufre associée à la matière organique des roches mères, qui est à l'origine du soufre présent dans le pétrole généré par ces roches mères. En particulier, la quantification distincte du soufre pyritique et du soufre organique permet de :

- caractériser le type de matière organique de la roche mère et prévoir la qualité du pétrole généré par la roche mère en ce qui concerne sa teneur en soufre : en effet, la caractérisation du type de matière organique des roches mères se fait classiquement en fonction du contenu élémentaire de cette matière organique en Carbone (C), en Hydrogène (H) et en Oxygène (O). Cette caractérisation classique du type de matière organique se fait à l'aide du diagramme classique de Van Krevelen, qui représente le rapport atomique Hydrogène/Carbone (H/C), en fonction du rapport atomique Oxygène/Carbone (O/C). Le potentiel d'une matière organique à générer du pétrole dépendant de sa composition en H, C et O, ce diagramme permet de distinguer trois types de matière organique selon leur potentiel pétrolier. En effet, ce diagramme peut être corrélé à l'origine et à l'environnement de dépôt de la matière organique. Classiquement, on distingue la matière organique de type lacustre (type I), marine (type II) et terrestre (type III). La quantification du soufre de la matière organique indépendamment du soufre de la pyrite (ou soufre pyritique) fournit un paramètre supplémentaire qui permet une plus fine caractérisation du type de matière organique et donc une plus fine caractérisation de son environnement de dépôt et du type de pétrole qu'elle peut générer. Cette caractérisation plus fine se fait avec le diagramme de Van Krevelen étendu à trois dimensions : H/C en fonction de O/C et de $S^{org}/C$ où $S^{org}$ est la teneur en soufre organique. Ce diagramme étendu permet de distinguer avec plus de finesse les différents types de matière organique, en particulier d'identifier les matières organiques de type IS et IIS qui ont les mêmes origines que les types I et II, mais contenant du soufre, et qui se sont probablement déposées en environnement anoxique ou euxinique. Cette présence de soufre indique aussi que le pétrole résultant du craquage de cette matière organique sera plus soufré. De manière générale, les informations sur le type de matière organique de la roche mère renseignent sur le potentiel de la roche mère à générer du pétrole et sur la qualité attendue de ce pétrole notamment en ce qui concerne sa teneur en soufre ;
- apporter un paramètre supplémentaire à la corrélation huile-roche mère : en effet, la corrélation huile-roche mère est une étude très importante que doit réaliser l'homme du métier pour évaluer le système pétrolier. Elle consiste à faire le lien entre les huiles contenues dans un réservoir et la ou les roches mères qui ont généré ces huiles. Sachant que le craquage des roches mères contenant de la matière organique riche en soufre aboutit à la formation d'huiles et de gaz aussi riches en soufre, une méthode de quantification du soufre de la matière organique, indépendamment du soufre de la pyrite, fournit donc un paramètre clé pour la corrélation huile-roche mère.

## État de la technique

On connaît notamment le brevet EP 2342557 (US 8796035) qui concerne un dispositif et une méthode pour la caractérisation et la quantification du soufre dans un échantillon de roches sédimentaires ou de produits pétroliers. Plus précisément, la méthode décrite dans ce brevet comprend les étapes suivantes :

- on chauffe l'échantillon considéré dans un four de pyrolyse en atmosphère non oxydante,
- on oxyde une partie des effluents de pyrolyse, et on mesure en continu la quantité de $SO_2$ contenue dans cette partie des effluents oxydés,
- on transfère les résidus de pyrolyse dans un four d'oxydation et on mesure en continu la quantité de $SO_2$ contenue dans les effluents résultants de la chauffe oxydante du résidu de pyrolyse,
- et on en déduit la teneur en soufre dans l'échantillon.

Toutefois, cette méthode permet de déterminer la teneur en soufre total présent dans l'échantillon étudié, mais ne permet pas de quantifier séparément le soufre pyritique du soufre organique. En effet, cette méthode permet de quantifier la teneur en soufre total d'un échantillon de roche, via la mesure des effluents soufrés libérés par cet échantillon au cours d'une pyrolyse puis d'une oxydation. Deux profils correspondant au soufre sont ainsi obtenus : le premier pendant la phase de pyrolyse, et le second pendant la phase d'oxydation. Au niveau du signal soufre de pyrolyse, il est possible de discriminer le soufre organique du soufre minéral dû à la pyrite, car ils forment systématiquement deux pics suffisamment distincts. Cependant, en oxydation, les signaux de ces deux composés soufrés sont confondus, ce qui empêche la spéciation du soufre organique et pyritique. De plus, de nombreuses réactions chimiques se produisent dans la roche au cours de l'analyse même. Si certaines impliquent le soufre organique et/ou le soufre pyritique, alors elles sont susceptibles de modifier leurs signaux, ce qui rajoute un niveau de difficulté à la quantification du soufre organique et du soufre pyritique au moyen du procédé tel que décrit dans le brevet précité.

Les documents suivants seront cités dans la suite de la description :

Acholla, F.V., Orr, W.L., 1993. Pyrite removal from kerogen without altering organic matter: The chromous chloride method. Energy Fuels 7, 406-410.

Bolin, T.B., 2010. Direct détermination of pyrite content in Argonne premium coals by the use of sulfur X-ray near edge absorption spectroscopy (S-XANES). Energy and Fuels 24, 5479-5482.

Canfield, D.E., Raiswell, R., Westrich, J.T., Reaves, C.M., Berner, R.A., 1986. The use of chromium reduction in the analysis of reduced inorganic sulfur in sediments and shales. Chemical Geology 54, 149-155.

Orr W., 1986, "Kerogen/asphaltene/sulfur relationships in sulfur-rich Monterey oils", Org. Geochem. Vol. 10, pp. 499-516, 1986.

Vairavamurthy, M.A., Maletic, D., Wang, S., Manowitz, B., Eglinton, T., Lyons, T., 1997. Characterization of sulfur-containing functional groups in sedimentary humic substances by X-ray absorption near-edge structure spectroscopy. Energy and Fuels 11, 546-553.

Vandenbroucke, M., Largeau, C., 2007. Kerogen origin, evolution and structure. Organic Geochemistry 38, 719-833.

On connaît des méthodes de laboratoire permettant de quantifier de manière distincte le soufre pyritique du soufre organique, telles que les techniques suivantes :

- <u>Analyse élémentaire des kérogènes</u>, telle que décrite dans le document (Vandenbroucke and Largeau, 2007). Il s'agit de l'une des méthodes les plus couramment utilisées en routine dans les laboratoires. Elle se déroule en 2 étapes :

  - Isolation du kérogène (ou encore isolation de la matière organique) : le kérogène est isolé de la roche brute par une série d'attaques chimiques à l'acide chlorhydrique et fluorhydrique visant à détruire la matrice minérale, les carbonates et les silicates. La pyrite ($FeS_2$), d'autres sulfures métalliques, ainsi que certains oxydes mineurs dont des oxydes de fer, étant résistants à ces différentes attaques chimiques, restent conservés dans le résidu organique obtenu. Ainsi on obtient un kérogène débarrassé de la matrice minérale mais contenant encore de la pyrite.
  - Analyse élémentaire du fer (par spectrométrie d'émission atomique avec plasma couplé par induction, connue sous le terme ICP-AES en anglais) et du soufre (par analyse infrarouge) : on fait ici l'hypothèse que le fer présent dans le kérogène obtenu, ne serait que sous forme de pyrite ($FeS_2$). Par conséquent à partir de la mesure de la teneur en fer (ICP-AES) du kérogène, la teneur en pyrite peut être calculée stoechiométriquement, permettant de déterminer ainsi la teneur en soufre pyritique. Puis à partir de la mesure de la teneur en soufre (IR) du kérogène on en déduit la teneur en soufre organique par différence entre le soufre total (mesuré par infrarouge) et le soufre pyritique.

  Cette première méthode de laboratoire selon l'art antérieur présente les inconvénients suivants :

  - la longueur du temps d'analyse : environ une semaine ;
  - elle nécessite des étapes de préparation, de séparation chimique, qui sont lourdes, dangereuses car utilisant des acides forts ;
  - elle ne permet pas l'automatisation des mesures ;
  - elle repose sur l'hypothèse que tout le fer contenu dans la matière organique est pyritique. Or si le fer contenu dans la matière organique se trouve aussi sous d'autres formes telles que des oxydes ou d'autres sulfures que $FeS_2$, alors on surestime la teneur en soufre pyritique et l'on sous-estime la teneur en soufre organique.

- <u>Extraction de la pyrite par le chlorure de chrome II et par l'analyse élémentaire de la roche de départ</u>, telle que décrit dans les documents (Canfield et al., 1986; Acholla et Orr, 1993). Selon cette approche, une attaque chimique à l'acide chlorhydrique (HCl) à chaud est réalisée dans un premier temps pour extraire tout le soufre volatile contenu dans des échantillons de roches. Une fois cette étape réalisée, les échantillons sont ensuite traités, à chaud, avec une solution constituée d'acide chlorhydrique (HCl) et de chlorure de chrome II ($CrCl_2$) permettant d'extraire la pyrite ($FeS_2$). L'effluent soufré ($H_2S$), libéré par la réduction de la pyrite par cette solution, passe dans un piège composé d'une solution de nitrate d'argent ($AgNO_3$), où il précipite sous forme de sulfure d'argent ($Ag_2S$). Le précipité d'$Ag_2S$ obtenu est pesé, ce qui permet de quantifier stoechiométriquement la teneur en soufre pyritique, en supposant que la pyrite a été entièrement transformée en sulfure d'argent. Puis la teneur en soufre organique est déduite par différence entre la teneur en soufre total, obtenue par l'analyse élémentaire de la roche de départ, et la teneur en soufre pyritique. Cette méthode repose sur l'hypothèse que toute la pyrite est réduite en $H_2S$.
  Cette deuxième méthode de laboratoire selon l'art antérieur présente les inconvénients suivants :

  - elle nécessite des étapes de préparation, de séparation chimique, qui sont lourdes, dangereuses car utilisant des acides forts ;
  - elle ne permet pas l'automatisation des mesures ;
  - elle repose sur l'hypothèse que toute la pyrite est réduite en $H_2S$. Si une partie de la pyrite n'est pas réduite, alors on sous-estime la teneur en soufre pyritique et l'on surestime la teneur en soufre organique. En particulier, les échantillons riches en pyrite risquent d'être dans ce cas.

- <u>Spectroscopie de structure près du front d'absorption de rayons X du soufre (S-XANES en anglais)</u>, telle que décrite dans les documents (Vairavamurthy et al., 1997; Bolin, 2010) : selon cette approche, le S-XANES renseigne sur l'état d'oxydation des composés soufrés. Dans une analyse typique, le spectre d'un échantillon est déconvolué avec diverses combinaisons linéaires des spectres de différents standards de soufre. Le meilleur ajustement est choisi pour indiquer la composition réelle des différents composés soufrés de cet échantillon. Cette technique permet donc quantitativement de déterminer le soufre pyritique, le soufre organique et les sulfates. Dans le cas d'analyse de roches, notons qu'un broyage très fin de l'échantillon est souvent nécessaire afin de mieux quantifier le soufre pyritique, dont le pic est atténué dans les échantillons non finement broyés.
  Cette troisième méthode de laboratoire selon l'art antérieur présente les inconvénients suivants :

  - elle nécessite un broyage très fin des échantillons
  - elle nécessite l'accès à un synchrotron, qui est un équipement très lourd et très coûteux.

La présente invention vise à pallier ces inconvénients. Ainsi, la présente invention concerne une méthode pour caractériser le soufre présent dans un échantillon de roche, en quantifiant de manière distincte le soufre pyritique et le soufre organique, et ce, de manière rapide, simple, et précise.

**Le procédé selon l'invention**

L'invention concerne un procédé pour la quantification du soufre pyritique dans un échantillon de roche sédimentaire, dans lequel on applique au moins les étapes suivantes :

A. on chauffe ledit échantillon en atmosphère inerte, entre une première température comprise entre 100°C et 320°C et une deuxième température comprise entre 600°C et 700°C, en suivant un premier gradient de température compris entre 1°C/min et 30°C/min ;

B. on oxyde en continu au moins une partie des effluents issus de ladite chauffe dudit échantillon en atmosphère inerte, on mesure en continu une première quantité de $SO_2$ libérée en fonction du temps de ladite chauffe en atmosphère inerte, et on détermine au moins une teneur en soufre de pyrolyse $S_{Pyrol}$ et une teneur en soufre pyritique de pyrolyse $S_{Pyrol}^{Pyrit}$ à partir de ladite première quantité de $SO_2$ ;

C. on chauffe en atmosphère oxydante le résidu dudit échantillon issu de ladite chauffe en atmosphère inerte entre une troisième température comprise entre 280°C et 320°C et une quatrième température supérieure ou égale à 800°C, en suivant un second gradient de température compris entre 1°C/min et 30°C/min ;

D. on mesure en continu une deuxième quantité de $SO_2$ libérée en fonction du temps de ladite chauffe en atmosphère oxydante, on détermine au moins une teneur en soufre d'oxydation $S_{Oxy}$ à partir de ladite deuxième quantité de $SO_2$, et on détermine au moins une teneur en soufre total $S_{Total}$ par la somme de ladite teneur en soufre de pyrolyse $S_{Pyrol}$ et de ladite teneur en soufre d'oxydation $S_{Oxy}$ ;

Selon l'invention, on détermine au moins une teneur en soufre pyritique $S^{Pyrit}$ contenu dans ledit échantillon à partir d'une formule du type :

$$S^{Pyrit} = p(\alpha, \beta, \gamma) . S_{Pyrol}^{Pyrit} \quad ,$$

où $p(\alpha,\beta,\gamma)$ est une fonction de pondération dépendant d'un paramètre $\alpha$ représentant une proportion dudit soufre pyritique de pyrolyse par rapport audit soufre total, d'un paramètre $\beta$ représentant un effet de la matrice minérale sur ladite proportion, d'un paramètre $\gamma$ représentant un effet de la matrice organique sur ladite proportion, les valeurs desdits paramètres étant prédéterminées.

Ladite fonction de pondération $p(\alpha,\beta,\gamma)$ s'écrit sous la forme :

$$p(\alpha, \beta, \gamma) = \frac{(1 + \beta + \gamma)}{\alpha}$$

Selon un mode de réalisation de l'invention, ledit échantillon est de type roche réservoir, et ladite première température peut être comprise entre 100°C et 200°C.

Selon un autre mode de réalisation de l'invention, ledit échantillon est de type roche mère, et ladite première température peut être comprise entre 280°C et 320°C.

Selon l'invention, le paramètre $\alpha$ est compris entre 0.40 et 0.46, et vaut préférentiellement 0.43.

Selon une variante de mise en œuvre de l'invention, ledit échantillon de roche est de type argile, et le paramètre $\beta$ peut être compris entre 0.04 et 0.7, et vaut préférentiellement 0.38.

Selon une autre variante de mise en œuvre de l'invention, ledit échantillon de roche est de type marne, et le paramètre $\beta$ peut être compris entre 0.7 et 0.9, et vaut préférentiellement 0.78.

Selon une variante alternative de mise en œuvre de l'invention, ledit échantillon de roche est de type calcaire, et le paramètre $\beta$ peut être compris entre 0.85 et 0.97, et vaut préférentiellement 0.9.

Selon l'invention, ledit échantillon de roche contient une matière organique d'origine lacustre et/ou marine, et lequel le paramètre $\gamma$ vaut 0, ou ledit échantillon de roche contient une matière organique d'origine terrestre, et le paramètre $\gamma$ peut être compris entre 0.23 et 0.29, et vaut préférentiellement 0.26.

Selon une variante de mise en œuvre de l'invention, ladite quatrième température est comprise entre 800°C et 900°C, et on peut déterminer une teneur en soufre organique $S^{Org}$ selon la formule :

$$S^{Org} = S_{Total} - S^{Pyrit} .$$

Selon une autre variante de mise en œuvre de l'invention, ladite quatrième température est supérieure à 1150°C, et préférentiellement inférieure 1250°C, et on peut déterminer en sus une teneur en soufres sulfates $S_{Oxy}^{Sulfa}$ à partir de la dite deuxième quantité de $SO_2$, et on peut en déduire une teneur en soufre organique selon la formule :

$$S^{Org} = S_{Total} - S^{Pyrit} - S_{Oxy}^{Sulfa} .$$

Selon une mise en œuvre de l'invention, on peut déterminer ladite teneur en soufre de pyrolyse et/ou ladite teneur en soufre pyritique de pyrolyse à partir de ladite première quantité de $SO_2$ et d'un coefficient de calibration du soufre de pyrolyse établi sur un échantillon de référence dont on connaît la teneur en soufre, ledit échantillon de référence étant de préférence du soufre natif.

Selon une mise en œuvre de l'invention, on peut déterminer ladite teneur en soufre d'oxydation à partir de ladite deuxième quantité de $SO_2$ et d'un coefficient de calibration du soufre d'oxydation établi sur un échantillon de référence dont on connaît la teneur en soufre, ledit échantillon de référence étant de préférence un charbon.

Avantageusement, on peut en outre mesurer :

- les quantités de produits hydrocarbonés, de CO et de $CO_2$ contenues dans lesdits effluents issus de ladite chauffe dudit échantillon en atmosphère inerte,
- les quantités de CO et de $CO_2$ contenues dans les effluents issus de ladite chauffe dans les effluents issus de ladite chauffe en atmosphère oxydante.

D'autres caractéristiques et avantages du procédé selon l'invention, apparaîtront à la lecture de la description ci-après d'exemples non limitatifs de réalisations, en se référant aux Figures annexées et décrites ci-après.

**Présentation succincte des figures**

- la Figure 1a présente un exemple de mesure réalisée par un détecteur de $SO_2$ au cours d'une séquence de chauffe sous atmosphère inerte à laquelle est soumise un échantillon de roche.

- la Figure 1b présente un exemple de mesure réalisée par un détecteur de $SO_2$ au cours d'une séquence de chauffe sous atmosphère oxydante à laquelle est soumise un échantillon de roche.

- la Figure 2 présente des courbes représentatives de la quantité de SO2 libérée par quatre échantillons de pyrite ignée pure de masses distinctes pendant une séquence de chauffe sous atmosphère inerte.

- La Figure 3a présente un histogramme représentatif de l'effet de la matrice minérale, en fonction de la classe de mélanges de minéraux considérée.

- La figure 3b présente un histogramme représentatif de l'effet moyen des argiles, des carbonates et des formations intermédiaires sur la proportion de soufre de la pyrite libérée pendant la pyrolyse en fonction de la classe de mélanges de minéraux considérée.

- La figure 3c présente l'évolution de l'effet de la matrice minérale en fonction du carbone minéral.

- La figure 4a présente une estimation de l'effet de la matrice organique sur la quantité de soufre libéré par la pyrite pendant la phase de pyrolyse en fonction d'une première série de classes de mélanges pyrite-matière organique.

- La figure 4b présente une estimation de l'effet de la matrice organique sur la quantité de soufre libéré par la pyrite pendant la phase de pyrolyse en fonction d'une deuxième série de classes de mélanges pyrite-matière organique.

- Les figures 5a, 5b, et 5c présentent respectivement l'évolution de la teneur en soufre total, en soufre pyritique et en soufre organique pour différents échantillons de roche obtenue par le procédé selon l'invention en fonction de la teneur en soufre total obtenue pour ces mêmes échantillons par une méthode selon l'art antérieur.

**Description détaillée du procédé**

De façon générale, l'un des objets de l'invention concerne un procédé pour quantifier de manière distincte le soufre pyritique du soufre organique présents dans un échantillon de roche.

La présente invention peut s'appliquer à tout type de roches sédimentaires, comme par exemple à une roche mère, à une roche réservoir ou bien à une roche mère non conventionnelle. En particulier, la présente invention est adaptée à des échantillons de roche provenant de roches mères marines.

De manière générale, l'échantillon de roche peut par exemple avoir été prélevé par carottage au sein d'une formation souterraine d'intérêt ou bien résulter de déblais issus d'un forage. Avantageusement, l'échantillon tel que prélevé est préparé (par un lavage, un tamisage, un triage, etc.) afin d'en éliminer les impuretés (boue de forage par exemple, polluants etc.), puis est broyé à la main ou avec un broyeur mécanique.

Le procédé selon l'invention repose sur la mesure du dioxyde de soufre ($SO_2$) libéré par un échantillon d'une roche soumis dans un premier temps à une pyrolyse (c'est-à-dire à une chauffe sous atmosphère inerte) puis à une oxydation (c'est-à-dire à une chauffe sous atmosphère oxydante).

Le procédé selon l'invention peut être avantageusement mais non limitativement mis en œuvre au moyen du dispositif ROCK-EVAL® (IFP Energies nouvelles, France), tel que décrit dans le brevet EP 2342557 (US 8796035).

Le procédé selon l'invention comporte au moins les étapes suivantes :

**EP 3 454 061 B1**

1. Séquence de chauffe sous atmosphère inerte (pyrolyse)
2. Séquence de chauffe sous atmosphère oxydante (oxydation)
3. Quantification du soufre pyritique

## 1. Séquence de chauffe sous atmosphère inerte (pyrolyse)

Au cours de cette étape, l'échantillon considéré est chauffé sous atmosphère inerte (comme par exemple sous un flux d'azote, d'hélium) selon un programme de températures prédéfinies, variables dans le temps.

Selon une mise en œuvre de l'invention, cette étape est réalisée au moyen d'un four à pyrolyse, l'échantillon d'intérêt placé dans ce four étant balayé par un flux de gaz non oxydant.

Selon l'invention, l'échantillon est chauffé par pyrolyse entre une température T1 comprise entre 100°C et 320°C, et une température T2 comprise entre 600°C et 700°C, préférentiellement 650°C, la hausse de température suivant un gradient de température (ou vitesse de chauffe) compris entre 1°C/min et 30°C/min, préférentiellement entre 20°C/min et 30°C/min, et vaut très préférentiellement 25°C/min. Selon une mise en œuvre de l'invention selon laquelle l'échantillon analysé est une roche réservoir, la température T1 est comprise entre 100° et 200°C, et est de 180°C préférentiellement. Selon une mise en œuvre de l'invention selon laquelle l'échantillon analysé est une roche mère, la température T1 est comprise entre 280° et 320°C, et est de 300°C préférentiellement.

Selon l'invention, au moins une partie des effluents de pyrolyse sont oxydés au fur et à mesure de leur libération. Les gaz soufrés présents dans les effluents de pyrolyse sont ainsi oxydés en $SO_2$. Selon une mise en œuvre de l'invention, cette oxydation des effluents de pyrolyse est réalisée au moyen d'une chambre de combustion, telle qu'un four d'oxydation, en présence d'un gaz oxygéné et éventuellement d'un catalyseur.

Selon l'invention, on mesure en continu, au fur et à mesure de la pyrolyse, le $SO_2$ ainsi généré, au moyen d'un détecteur de SO2 tel qu'un spectrophotomètre à ultra-violet (UV) ou à infra-rouge (IR). On obtient ainsi une mesure du $SO_2$ libéré au cours de la pyrolyse, en fonction du temps et/ou de la température de pyrolyse.

## 2. Séquence de chauffe en atmosphère oxydante (oxydation)

Au cours de cette deuxième étape, le résidu solide de l'échantillon obtenu à l'issue de la séquence de pyrolyse telle que décrite à l'étape 1 ci-dessus est soumis à une oxydation selon un programme de températures prédéfinies, variables dans le temps.

Selon l'invention, l'échantillon est chauffé sous atmosphère oxydante entre une température T3 comprise entre 280°C et 320°C, préférentiellement 300°C, et une température T4 supérieure ou égale à 800°C, la hausse de température suivant un gradient de température (ou vitesse de chauffe) compris entre 1°C/min et 30°C/min, préférentiellement entre 20°C/min et 30°C/min, et vaut très préférentiellement 20°C/min.

Selon une mise en œuvre de l'invention, cette étape est réalisée au moyen d'un four d'oxydation, le résidu de pyrolyse étant balayé par un flux d'air.

Selon l'invention, on mesure en continu, c'est-à-dire pendant la séquence de chauffe sous atmosphère oxydante, le $SO_2$ généré par l'oxydation du résidu de pyrolyse et contenu dans les effluents d'oxydation. La mesure de $SO_2$ est par exemple réalisée au moyen d'un spectrophotomètre à UV ou IR. On obtient ainsi une mesure du $SO_2$ libéré au cours de l'oxydation, par exemple en fonction du temps et/ou de la température d'oxydation.

## 3- Quantification du soufre pyritique

A l'issue des deux étapes précédentes, on dispose de deux courbes représentatives des mesures du $SO_2$ réalisées au cours des étapes 1 et 2 telles que décrites ci-dessus.

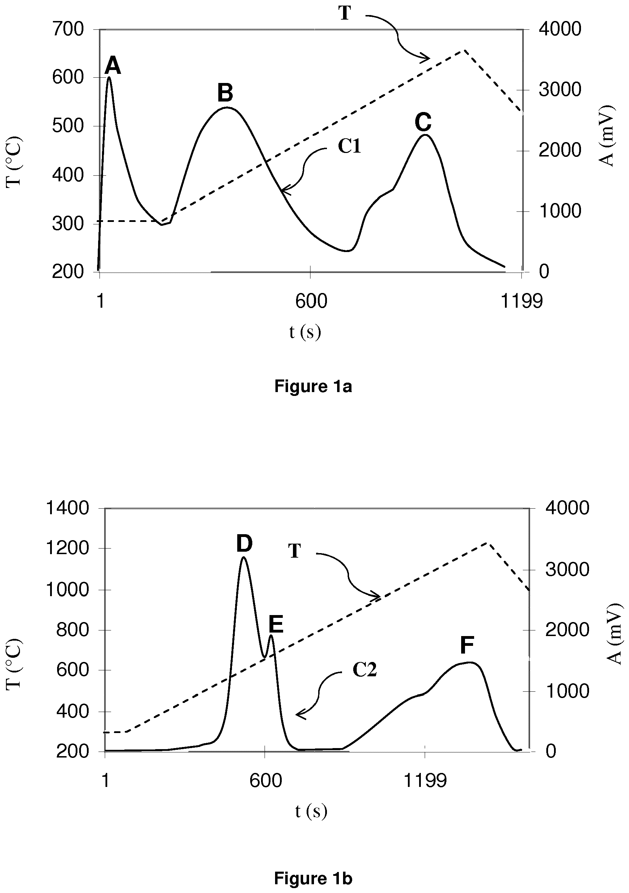

La figure 1A présente un exemple de courbe (notée C1) de mesure de la quantité de SO2 (plus précisément l'amplitude A mesurée par un détecteur de $SO_2$, tel qu'un spectrophotomètre à ultra-violet) en fonction du temps de pyrolyse (noté t), et présente également l'évolution de la température de pyrolyse (notée T) en fonction du temps de pyrolyse. La figure 1B présente un exemple de courbe (notée C2) de mesure de la quantité de SO2 (plus précisément l'amplitude A mesurée par un détecteur de $SO_2$, tel qu'un spectrophotomètre à ultra-violet) en fonction du temps d'oxydation (noté t), et présente également l'évolution de la température de d'oxydation (notée T) en fonction du temps d'oxydation. Pour cet exemple et à des fins illustratives, la température T1 a été choisie égale à 300°C, la température T2 a été choisie égale à 650°C, la température T3 a été choisie égale à 300°C et la température T4 a été choisie égale à 1200°C.

On peut observer que chacune de ces courbes comporte différents pics et est identifiable par le nombre de ces pics, leur température de sommet de pic, leur forme et leur aire. Notamment on peut observer sur la courbe C1 le pic C qui correspond à la libération durant la pyrolyse d'une partie du soufre contenu dans la pyrite (dit « soufre pyritique de pyrolyse » par la suite, et noté $S_{Pyrol}^{Pyrit}$). Sur la courbe C2, on peut observer le pic F qui correspond à la libération du soufre contenu dans les sulfates (dit « soufre sulfates » par la suite, et noté $S_{Oxy}^{Sulfa}$) durant l'oxydation. Par ailleurs, les deux premiers pics A et B de la courbe C1 correspondent au soufre contenu dans les composés organiques thermiquement labiles, qui sont respectivement vaporisables et thermiquement craquables. Également, on peut observer que la courbe C2 présente deux premiers pics D et E quasi-confondus, qui correspondent respectivement à du soufre organique contenu dans des composés organiques, qui sont thermiquement réfractaires ou bien qui ont été générés pendant la phase de pyrolyse, et à du soufre pyritique. On constate ainsi que l'enregistrement du $SO_2$ libéré pendant la phase d'oxydation ne permet pas de faire la distinction entre ces deux pics et donc entre le soufre organique et le soufre pyritique.

Au cours de cette étape, selon l'invention, on quantifie la teneur en soufre de pyrolyse $S_{Pyrol}$ libéré au cours de la pyrolyse, la teneur en soufre d'oxydation $S_{Oxy}$ libéré au cours de l'oxydation du résidu de pyrolyse, et la teneur en soufre pyritique de pyrolyse $S_{Pyrol}^{Pyrit}$ libéré au cours de la pyrolyse, à partir des mesures réalisées pendant la séquence de chauffe en atmosphère inerte et la séquence de chauffe en atmosphère oxydante.

Selon une mise en œuvre de l'invention, on peut déterminer la teneur en soufre de pyrolyse $S_{Pyrol}$ (respectivement la teneur en soufre d'oxydation $S_{Oxy}$) de l'échantillon analysé à partir de l'aire sous la courbe de mesure du $SO_2$ enregistrée pendant la séquence de chauffe par pyrolyse (respectivement pendant la séquence de chauffe oxydante), divisée par la masse de l'échantillon analysé, pondérée par un coefficient de calibration du soufre de pyrolyse (respectivement un coefficient de calibration du soufre d'oxydation). Ces teneurs sont exprimées en pourcentage massique, soit en masse de soufre de pyrolyse (respectivement d'oxydation), divisée par la masse de l'échantillon et multipliée par 100.

Selon une mise en œuvre de l'invention, on peut déterminer la teneur en soufre pyritique de pyrolyse $S_{Pyrol}^{Pyrit}$ à partir de l'aire sous le pic représentatif du soufre pyritique de pyrolyse sur la courbe de mesure du $SO_2$ enregistrée pendant la phase de pyrolyse (cf. pic C sur la Figure 1a), divisée par la masse de l'échantillon analysé, pondérée par un coefficient de calibration du soufre de pyrolyse. La teneur en soufre pyritique de pyrolyse est exprimée en pourcentage massique, soit en masse de soufre pyritique de pyrolyse, divisée par la masse de l'échantillon et multipliée par 100.

Selon une mise en œuvre de l'invention, on peut déterminer un coefficient de calibration du soufre de pyrolyse (respectivement un coefficient de calibration du soufre d'oxydation) à partir au moins d'un échantillon de référence dont on connaît la teneur en soufre, échantillon que l'on soumet à une séquence de chauffe par pyrolyse (respectivement une séquence de chauffe oxydante). Puis on détermine le coefficient de calibration du soufre de pyrolyse à partir de l'aire sous la courbe de mesure du $SO_2$ libéré par cet échantillon de référence pendant une séquence de chauffe par pyrolyse (respectivement pendant une séquence de chauffe oxydante), elle-même divisée par la masse de l'échantillon de référence. Selon une mise en œuvre de l'invention, l'échantillon de référence peut être du soufre natif pour la détermination du coefficient de calibration du soufre de pyrolyse. Selon une mise en œuvre de l'invention, l'échantillon de référence peut être un charbon pour la détermination du coefficient de calibration du soufre d'oxydation.

Selon l'invention, on détermine en outre la teneur en soufre total, $S_{Total}$ comme la somme des deux teneurs $S_{Pyrol}$ et $S_{Oxy}$, soit :

$$S_{Total} = S_{Pyrol} + S_{Oxy} \, ,$$

exprimée en pourcentage massique (wt.%), soit en masse de soufre total divisée par la masse de l'échantillon et multipliée par 100.

Selon l'invention, on détermine la teneur en soufre pyritique $S^{Pyrit}$ selon une formule du type :

$$S^{Pyrit} = p(\alpha, \beta, \gamma) . S_{Pyrol}^{Pyrit}$$

exprimée en pourcentage massique, soit en masse de soufre pyritique divisée par la masse de l'échantillon et multipliée par 100, $p(\alpha,\beta,\gamma)$ étant une fonction de pondération dépendant des paramètres $\alpha$, $\beta$ et $\gamma$, ces paramètres ayant été préalablement déterminés, avec :

- le paramètre $\alpha$, qui représente la proportion du soufre pyritique libérée pendant la phase de pyrolyse par rapport à son soufre total, et peut être vu comme un taux de dégradation thermique de la pyrite. Selon une mise en œuvre de l'invention, le paramètre $\alpha$ est compris entre 0.40 et 0.46, et vaut préférentiellement 0.43 ;

- le paramètre $\beta$, qui représente l'impact de la matrice minérale sur la proportion du soufre pyritique libérée pendant la phase de pyrolyse. En effet, la matrice minérale réduit la quantité de soufre de la pyrite libérée pendant la phase de pyrolyse. Selon un aspect de l'invention, le paramètre β peut être compris entre 0.04 et 0.97, en fonction du type de roche dont provient l'échantillon étudié. Selon une mise en œuvre de l'invention dans laquelle l'échantillon de roche étudié est de type argile, le paramètre $\beta$ peut être compris entre 0.04 et 0.7, et vaut préférentiellement 0.38. Selon une mise en œuvre de l'invention dans laquelle l'échantillon de roche étudié est de type marne, le paramètre $\beta$ peut être compris entre 0.7 et 0.9, et vaut préférentiellement 0.78. Selon une mise en œuvre de l'invention dans laquelle l'échantillon de roche étudié est de type calcaire, le paramètre $\beta$ peut être compris entre 0.85 et 0.97, et vaut préférentiellement 0.90.
- le paramètre $\gamma$, qui représente l'impact de la matrice organique sur la proportion du soufre pyritique libérée pendant la phase de pyrolyse. Selon une mise en œuvre de l'invention, le paramètre $\gamma$ peut être compris entre 0 et 0.29, en fonction du type de matière organique. Selon une mise en œuvre de l'invention dans laquelle la matière organique présente dans l'échantillon de roche étudié est de type marin ou lacustre, le paramètre $\gamma$ vaut 0 (aucun effet significatif sur la dégradation de la pyrite pendant la phase de pyrolyse). Selon une mise en œuvre de l'invention dans laquelle la matière organique présente dans l'échantillon de roche étudié est de type terrestre, le paramètre $\gamma$ peut être compris entre 0.23 et 0.29, et vaut préférentiellement 0.26.

Selon une mise en œuvre de l'invention, la fonction de pondération $p(\alpha,\beta,\gamma)$ peut s'écrire sous la forme :

$$p(\alpha, \beta, \gamma) = \frac{(1+\beta+\gamma)}{\alpha}.$$

### 4- Quantification du soufre organique

Au cours de cette étape, qui est optionnelle, on peut déterminer la teneur en soufre organique $S^{Org}$ contenu dans l'échantillon de roche considéré à partir au moins de la différence entre la teneur en soufre total $S_{Total}$ et la teneur en soufre pyritique $S^{Pyrit}$.

Selon une première variante de mise en œuvre de l'invention selon laquelle la température de fin d'oxydation T4 est comprise entre 800°C et 900°C, on peut déterminer la teneur en soufre organique $S^{Org}$ contenue dans ledit échantillon selon une formule du type :

$$S^{Org} = S_{Total} - S^{Pyrit}$$

Selon une deuxième variante de mise en œuvre de l'invention selon laquelle la température de fin d'oxydation T4 est comprise entre 1150°C et 1250°C, préférentiellement 1200°C , on peut déterminer la teneur en soufre organique $S^{Org}$ contenu dans l'échantillon de la manière suivante :

- on quantifie une teneur en soufres sulfates $S^{Sulfa}_{Oxy}$ à partir de l'aire sous le pic représentatif du soufre sulfates de la courbe de mesure du $SO_2$ enregistrée pendant la phase d'oxydation, divisée par la masse de l'échantillon analysé, et pondérée par un coefficient de calibration du soufre d'oxydation (cf étape 3 ci-dessus pour la détermination de ce coefficient de calibration) ;
- on détermine la teneur en soufre organique $S^{Org}$ selon une formule du type :

$$S^{Org} = S_{Total} - S^{Pyrit} - S^{Sulfa}_{Oxy}.$$

En effet, pour cette variante de mise en œuvre, on peut distinguer le pic $S^{Sulfa}_{Oxy}$ (cf. pic F sur la Figure 1a) qui correspond à la libération durant l'oxydation du soufre contenu dans les sulfates, survenant pour les hautes températures. La détermination de la teneur en soufre organique est plus précise selon ce deuxième mode de mise en œuvre de l'invention.

### 5 - Calibration des paramètres $\alpha$, $\beta$ et $\gamma$

Selon une mise en œuvre de l'invention, on peut calibrer les paramètres $\alpha$ et/ou $\beta$ et/ou $\gamma$ tels que définis ci-dessus préalablement à la mise en œuvre du procédé selon l'invention, ou bien au cours de la mise en œuvre du procédé selon l'invention, par exemple préalablement à l'étape 1, à l'étape 2 ou à l'étape 3 décrites ci-dessus.

• **calibration du paramètre** $\alpha$

Selon une mise en œuvre de l'invention, on peut calibrer le paramètre $\alpha$ en estimant la proportion du soufre pyritique libéré pendant la phase de pyrolyse par rapport au soufre total à partir d'au moins un échantillon de pyrite ignée pure. Selon une mise en œuvre de l'invention, on peut obtenir une pyrite dite pure en nettoyant une pyrite naturelle de ces impuretés par des attaques chimiques.

Un exemple de calibration du paramètre $\alpha$ est décrit ci-après. Quatre échantillons issus d'un unique échantillon de pyrite ignée pure (notés respectivement E1, E2, E3, E4), de masses différentes (respectivement 2mg, 3mg, 4mg et 8mg) sont chacun soumis à une pyrolyse au moyen du dispositif ROCK-EVAL® (IFP Energies nouvelles, France). Notamment, pour cet exemple de calibration du paramètre $\alpha$, chaque échantillon a été placé dans le four à pyrolyse du dispositif ROCK-EVAL®, puis on a procédé à un chauffage de l'échantillon entre 300°C et 650°C, avec une rampe de température de 25°C/min et sous un flux d'azote à 150ml/min. Puis, les effluents soufrés libérés par chaque échantillon de pyrite ignée pure considéré ont été entrainés par le flux d'azote vers la chambre de combustion (four d'oxydation) du dispositif ROCK-EVAL®, où ils ont été transformés en $SO_2$ en flux continu, puis le $SO_2$ a été entraîné jusqu'à un détecteur de $SO_2$ où il a été quantifié en continu au moyen du détecteur de $SO_2$ du dispositif ROCK-EVAL®. Le résidu solide de chaque échantillon de pyrite ignée, obtenu à l'issue de la séquence de pyrolyse, a été ensuite placé dans le four à oxydation du dispositif ROCK-EVAL® puis on a procédé à un chauffage de l'échantillon entre 300°C et 850°C, avec une rampe de température de 20°C/min et sous un flux d'air à 100ml/min. Les effluents $SO_2$ libérés ont été entraînés jusqu'à un détecteur de $SO_2$ où ils ont été quantifiés en continu au moyen du détecteur de $SO_2$ du dispositif ROCK-EVAL®.

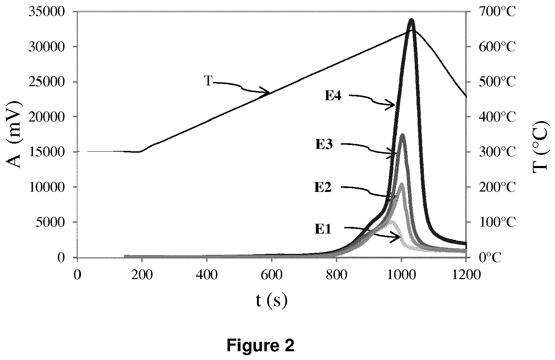

La figure 2 présente l'enregistrement dans le temps t de la quantité de $SO_2$ (plus précisément l'amplitude libéré par les échantillons E1, E2, E3, et E4 pendant la phase de pyrolyse telle que décrite ci-dessus. La courbe T présentée également sur cette figure 2 correspond à l'évolution de la température à laquelle est soumis chacun des échantillons considérés pendant cette même phase de pyrolyse. On peut notamment observer sur cette figure la présence des pics, représentatifs de la dégradation thermique de la pyrite aux différentes masses analysées pendant la phase de la pyrolyse. La teneur en soufre de pyrolyse de l'échantillon de pyrite ignée (teneur en soufre pyritique de pyrolyse) a été calculée en multipliant par la teneur en soufre de l'échantillon de référence l'aire sous chacune des courbes E1, E2, E3 et E4, divisée par la masse de l'échantillon, et ramenée à l'aire sous la courbe de mesure du $SO_2$ libéré par un échantillon de référence (tel que du soufre natif) pendant la séquence de chauffe par pyrolyse, elle-même divisée par la masse de l'échantillon de référence. Le ratio entre cette teneur en soufre pyritique de pyrolyse et la teneur en soufre total de la pyrite (décrite dans l'étape 3 ci-dessus) est calculé. Les résultats montrent que, quelle que soit la masse analysée, la proportion massique du soufre pyritique qui est libérée pendant la pyrolyse est de 0.43 $\pm$ 0.03 %wt. La proportion restante de soufre pyritique à l'issue de la pyrolyse (0.57 $\pm$ 0.03 %wt) est libérée ensuite pendant la phase d'oxydation (étape 2 décrite ci-dessus).

Ainsi, la calibration telle que décrite ci-dessus permet de déterminer que le paramètre $\alpha$ est compris entre 0.40 et 0.46, et vaut 0.43 en moyenne.

• **calibration du paramètre** $\beta$

Selon une mise en œuvre de l'invention, on calibre le paramètre $\beta$ qui représente l'impact de la matrice minérale sur la quantité du soufre de la pyrite libérée pendant la phase de pyrolyse à partir d'au moins un mélange de pyrite et d'au moins un type de minéral, ce mélange étant représentatif de l'échantillon de roche à étudier par le procédé selon l'invention.

Un exemple de calibration du paramètre $\beta$ pour différents types de minéraux est décrit ci-après. Pour cet exemple de calibration du paramètre $\beta$, on a réalisé des mélanges à partir des deux grands groupes de minéraux suivants :

-   des minéraux argileux/silicatés, tels que de la

    • Silice (Sable de Fontainebleau, France) le mélange réalisé avec la silice est le mélange de référence car la silice est connu pour être non réactive ;
    • Kaolinite (Reference : CMS KGa 1b) ;
    • Smectite (Reference : Mx80) ;
    • Illite (Argile du Velay, France) : cet échantillon contenant naturellement des carbonates, il a été décarbonaté avec de l'acide chlorhydrique.

-   des minéraux carbonatés, tels que de la :

- Calcite (France) ;
- Dolomite (Euguy, Espagne) ;
- Sidérite (Pérou).

On réalise alors les mélanges suivants :

- 2mg de pyrite + 98mg de chaque minéral argileux/silicaté ;
- 2mg de pyrite + 58mg de chaque minéral carbonaté ;
- 2mg de pyrite + 98mg d'argiles (tous les minéraux argileux/silicatés à part égale ¼ ; ¼ ; ¼ ; ¼)) ;
- 2mg de pyrite + 58mg de carbonates (tous les minéraux carbonatés à part égale 1/3 ; 1/3 ; 1/3) ;
- 2mg de pyrite + 58mg d'argiles et de carbonates à différentes proportions, soit

  • 93% d'argiles et 7% de carbonates ;
  • 69% d'argiles et 31% de carbonates ;
  • 51 % d'argiles et 49% de carbonates ;
  • 26% d'argiles et 74% de carbonates.

Ces différents échantillons sont alors soumis aux étapes 1 et 2 telles que décrites ci-dessus au moyen du dispositif ROCK-EVAL® (IFP Energies nouvelles, France). Plus précisément, chaque échantillon est placé dans le four à pyrolyse du dispositif ROCK-EVAL® puis on procède à un chauffage de l'échantillon entre 300°C et 650°C, avec une rampe de température de 25°C/min et sous un flux d'azote à 150ml/min. Selon une mise en œuvre de l'invention, les effluents soufrés libérés par chaque échantillon sont entraînés par un flux d'azote vers la chambre de combustion (four d'oxydation) du dispositif ROCK-EVAL® où ils sont transformés en $SO_2$ en flux continu, puis le $SO_2$ est entraîné jusqu'au détecteur de $SO_2$ du dispositif ROCK-EVAL® où il est quantifié en continu. Le résidu solide de chaque échantillon obtenu à l'issu de la séquence de pyrolyse est ensuite placé dans le four à oxydation du dispositif ROCK-EVAL® puis on procède à un chauffage de l'échantillon entre 300°C et 850°C, avec une rampe de température de 20°C/min et sous un flux d'air à 100ml/min. Les effluents $SO_2$ libérés sont entraînés jusqu'à un détecteur de $SO_2$ où ils sont quantifiés en continu au moyen du détecteur de $SO_2$ du dispositif ROCK-EVAL®.

Par la suite, on appelle « effet de la matrice minérale » la grandeur s'exprimant selon une formule du type :

$$E_{Min} = \frac{S_{Pyrol}^{Pyrit,ref} - S_{pyrol}^{Pyrit,Matrix}}{S_{Pyrol}^{Pyrit,ref}} * 100,$$

où $S_{Pyrol}^{Pyrit,ref}$ est le soufre pyritique de pyrolyse libéré par un échantillon de référence (constitué de pyrite ignée pure et de silice) et $S_{pyrol}^{Pyrit,Matrix}$ est le soufre pyritique de pyrolyse libéré par un mélange considéré (pyrite ignée pure plus un minéral ou un mélange de minéraux). Pour évaluer cette grandeur, on détermine la teneur en soufre pyritique de pyrolyse tel que décrit à l'étape 3 ci-dessus et ce, pour un échantillon de référence $S_{Pyrol}^{Pyrit,ref}$ et pour un mélange considéré $S_{Pyrol}^{Pyrit,Matrix}$.

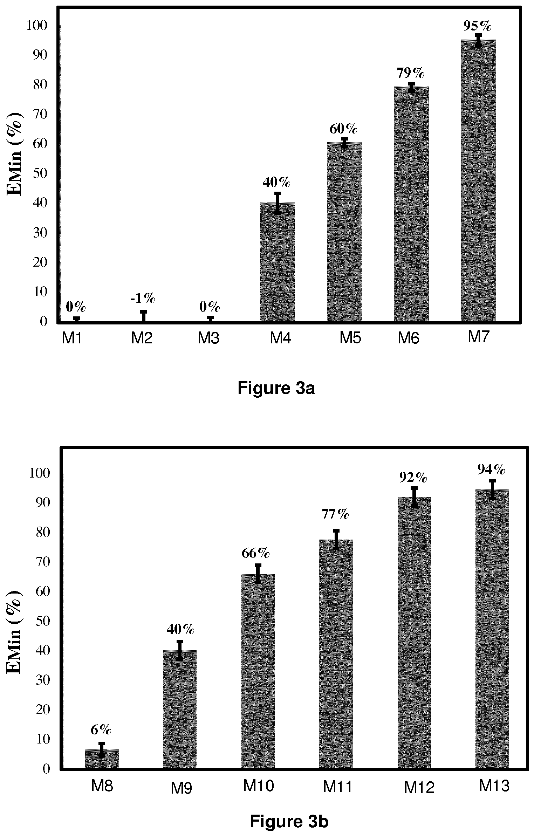

La figure 3a présente un histogramme représentatif de l'effet $E_{Min}$ de la matrice minérale en fonction de la classe de mélanges considérés dans le cas de minéraux argileux/silicatés et carbonatés, plus précisément pour les classes de mélanges suivantes :

- M1 : mélanges constitués de pyrite et de quartz (échantillon de référence) ;
- M2 : mélanges constitués de pyrite et kaolinite ;
- M3 : mélanges constitués de pyrite et d'illite ;
- M4 : mélanges constitués de pyrite et de smectite ;
- M5 : mélanges constitués de pyrite et de calcite ;
- M6 : mélanges constitués de pyrite et de dolomite ;
- M7 : mélanges constitués de pyrite et de sidérite.

La figure 3b présente un histogramme représentatif de l'effet $E_{Min}$ moyen des argiles, des carbonates et des formations intermédiaires sur la proportion de soufre de la pyrite libérée pendant la pyrolyse pour les mélanges suivants :

- M8 : mélanges constitués de 100% d'argiles ;
- M9 : mélanges constitués de 93% d'argiles et de 7% de carbonates ;
- M10 : mélanges constitués de 69% d'argiles et de 31% de carbonates ;
- M11 : mélanges constitués de 51% d'argiles et de 49% de carbonates ;
- M12 : mélanges constitués de 26% d'argiles et de 74% de carbonates ;
- M13 : mélanges constitués de 100% de carbonates.

Les figures 3a et 3b présentent aussi les barres d'erreurs pour chaque barre d'histogramme. Ces barres d'erreur ont été obtenues en estimant un écart-type établi à partir d'une répétition des analyses telles que décrites ci-dessus.

Ainsi, les résultats obtenus par la mise en œuvre de la méthode de calibration du paramètre β telle que décrite ci-dessus pour les différents mélanges décrits ci-dessus mettent en évidence le fait que la matrice minérale peut réduire la proportion de soufre de la pyrite libérée pendant la phase de pyrolyse. Toutefois, cet effet est très variable selon type de minéral en présence. La réduction relative de la proportion de soufre libérée par la pyrite en pyrolyse varie entre 0% et 40% en présence de minéraux argileux/silicatés et entre 60% et 98% en présence de minéraux carbonatés (cf. figure 3a). L'effet moyen des argiles s'élève à 6% tandis que celui des carbonates atteint 93% (cf. figure 3b). Entre ces deux pôles on observe une évolution croissante de l'effet de la matrice $E_M$ en fonction de la part d'argiles et de carbonates dans le mélange (cf. figure 3b).

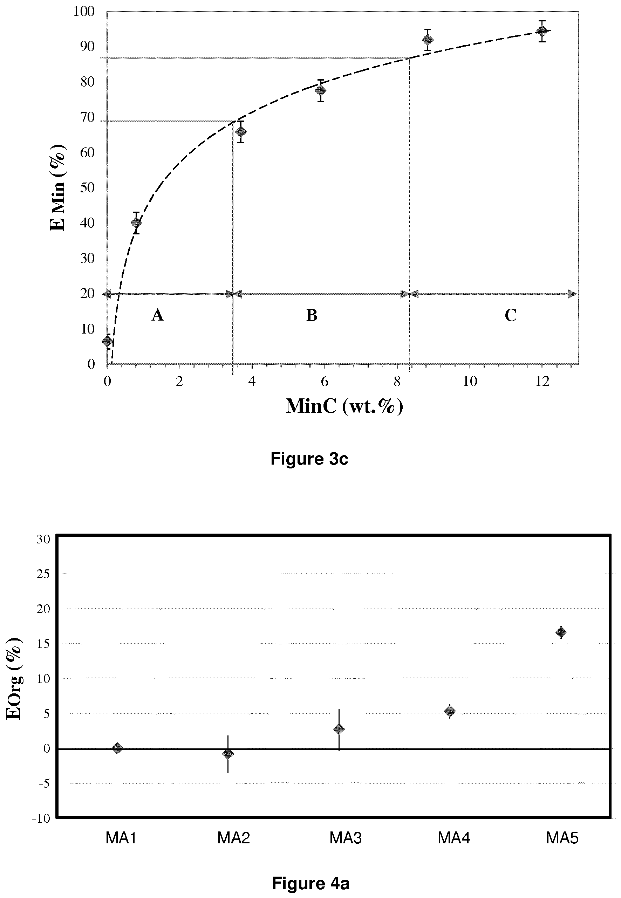

La figure 3c montre l'évolution de l'effet $E_{Min}$ de la matrice minérale en fonction du carbone minéral (noté MinC ci-après), qui est un paramètre pouvant être mesuré par exemple avec le dispositif ROCK-EVAL® (IFP Energies nouvelles, France), et qui est un indicateur de la teneur en carbonates des mélanges. On peut observer sur cette figure que le MinC varie sur une gamme comprise entre 0wt% et 12wt%, ce qui correspond à un équivalent calcite entre 0wt% et 100wt%. Grâce à ce paramètre, on peut définir trois types de lithologies : les argiles, les marnes et les calcaires. La zone (A) de la figure 3c représente la zone des argiles, qui ont des teneurs en carbonates-équivalent calcite comprises entre 0wt% et 30wt% (0≤MinC argiles<3.6wt%). Dans cette zone des formations argileuses, l'effet de la matrice sur la quantité du soufre de la pyrite libérée pendant la phase de pyrolyse varie entre 6% et 70%, avec une moyenne de 38%. La zone (B) de la figure 3c représente la zone des marnes, qui ont des teneurs en carbonates-équivalent calcite comprises entre 30% et 70% (3.6≤MinC marnes<8.4wt%). Dans cette zone des formations marneuses, la valeur moyenne d'effet de la matrice sur la quantité du soufre de la pyrite libérée pendant la phase de pyrolyse varie entre 70% et 87%, avec une moyenne de 78%. La zone (C) de la figure 3c représente la zone des calcaires qui ont des teneurs en carbonates-équivalent calcite comprises entre 70% et 100% (8.4wt%≤MinC calcaires≤12wt%). Dans cette zone des formations calcaires, la valeur moyenne d'effet de la matrice sur la quantité du soufre de la pyrite libérée pendant la phase de pyrolyse varie entre 87% et 94%, avec une moyenne de 90%.

Ainsi, le paramètre β varie entre 0.06 et 0.94 selon le type de formations sédimentaires, et plus précisément, dans le cas des :

- Argiles : le paramètre β vaut en moyenne 0.38 ;
- Marnes : le paramètre β vaut en moyenne 0.78 ;
- Calcaires : le paramètre β vaut en moyenne 0.90.

**• calibration du paramètre γ**

Selon une mise en œuvre de l'invention, on calibre le paramètre γ qui représente l'impact de la matrice organique sur la quantité de soufre libéré par la pyrite pendant la phase de pyrolyse à partir d'au moins un mélange constitué de pyrite et de matière organique représentative de celle présente dans l'échantillon de roche à étudier. Dans les échantillons de roches naturelles, notamment dans les roches mères et dans les roches réservoir, la pyrite se trouve en présence de matière organique.

Un exemple de calibration du paramètre γ est décrit ci-après.

Selon une mise en œuvre de l'invention comprenant une étape de calibration du paramètre γ pour différents types de matière organique, on réalise des mélanges constitués de pyrite et de différents types de matière organique classiquement notés :

- Type I : matière organique lacustre, telle que les « green river shales » (Eocène, USA) ;
- Type II: matière organique marine, telle que les « schistes carton » du Bassin de Paris (Toarcien, France);
- Type IIS : matière organique marine riche en soufre organique, telle que le « Phosphoria Formation » (Permien, USA) ;
- Type III : matière organique terrestre, telle que le « Calvert bluff Formation » (Paleocène, USA);

Selon une mise en œuvre de l'invention, on peut réaliser des mélanges tels que :

- Mélange de type A : 2mg de pyrite + 2mg de matière organique;
- Mélange de type B : 2mg de pyrite + 4mg de matière organique.

Ces mélanges sont représentatifs d'une composition type des kérogènes des formations sédimentaires.

Par la suite, on appelle « effet de la matrice organique » la grandeur s'exprimant selon la formule suivante :

$$:E_{Org} = \frac{S_{Pyrol}^{Pyrit+MO\ obtenu} - S_{pyrol}^{Pyrit+MO\ attendu}}{S_{Pyrol}^{Pyrit+MO\ attendu}} \times 100 \,, \quad \text{où} \quad S_{Pyrol}^{Pyrit+MO\ obtenu}$$ est le soufre pyritique de pyrolyse obtenu après l'analyse du mélange formé de pyrite et de matière organique (telle que décrite à l'étape 3) et $S_{Pyrol}^{Pyrit+MO\ attendu}$ est la valeur attendue de soufre pyritique de pyrolyse du mélange. Cette valeur de référence théorique est calculée comme suit :

- on analyse chaque échantillon de matière organique seul, au moyen du dispositif ROCK-EVAL® (IFP Energies nouvelles, France), pour quantifier sa teneur en soufre pyritique de pyrolyse (tel que décrite à l'étape 3) ;
- on analyse la pyrite seule, au moyen du dispositif ROCK-EVAL® (IFP Energies nouvelles, France), pour quantifier sa teneur en soufre pyritique de pyrolyse (tel que décrite à l'étape 3) ;
- on additionne proportionnellement, en fonction du ratio pyrite/matière organique, le soufre pyritique de pyrolyse de la pyrite et le soufre pyritique de pyrolyse de la matière organique.

La figure 4a présente l'effet $E_{Org}$ de la matrice organique sur la quantité de soufre libéré par la pyrite pendant la phase de pyrolyse pour les mélanges de type A, avec :

- MA1 : mélange constitué de 100% de pyrite ;
- MA2 : mélange constitué de 50% de pyrite et de 50 % de matière organique de type I ;
- MA3 : mélange constitué de 50% de pyrite et de 50 % de matière organique de type II ;
- MA4 : mélange constitué de 50% de pyrite et de 50 % de matière organique de type IIS ;
- MA5 : mélange constitué de 50% de pyrite et de 50 % de matière organique de type III ;

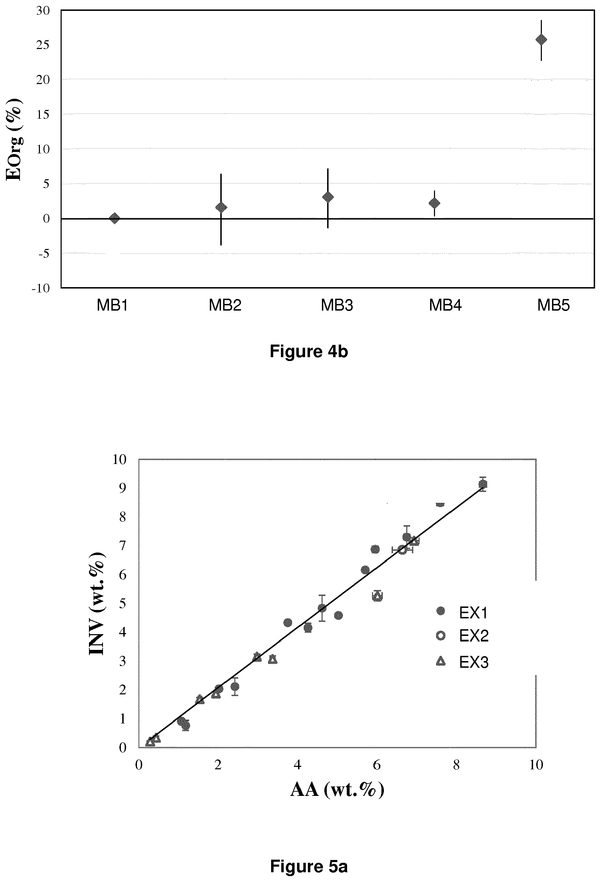

La figure 4b présente l'effet $E_{Org}$ de la matrice organique sur la quantité de soufre libéré par la pyrite pendant la phase de pyrolyse pour les mélanges de type B, avec

- MB1 : mélange constitué de 100% de pyrite ;
- MB2 : mélange constitué de 30% de pyrite et de 70 % de matière organique de type I ;
- MB3 : mélange constitué de 30% de pyrite et de 70 % de matière organique de type II ;
- MB4 : mélange constitué de 30% de pyrite et de 70 % de matière organique de type IIS ;
- MB5 : mélange constitué de 30% de pyrite et de 70 % de matière organique de type III ;

Les résultats obtenus par la mise en œuvre de la méthode de calibration du paramètre $\gamma$ telle que décrite ci-dessus pour les différents mélanges décrits ci-dessus montrent qu'il n'y a quasiment pas d'effet de la matière organique en ce qui concerne les type I, II et IIS. En effet, l'effet de la matière organique est inférieur à 6% pour ce type de mélanges (cf. figures 4a et 4b). Cependant, la matière organique de type III semble avoir un effet significatif sur la quantité du soufre de la pyrite libérée pendant la phase de pyrolyse (cf. figures 4a et 4b). En effet, l'effet de la matière organique de type III est de 17% en moyenne dans le mélange MA5, et de 26% dans le mélange MB5, mélange dont la proportion entre la pyrite et la matière organique est la plus représentative de celle des kérogènes de la majorité des formations sédimentaires (cf. figures 4a et 4b).

Ainsi, le paramètre $\gamma$ varie sur une gamme entre 0 et 0.29 selon le type de matière organique en présence, et plus précisément, dans le cas d'une matière organique :

- de type I, II ou IIS, $\gamma$ vaut 0 (figures 4a et 4b);
- de type III, $\gamma$ est compris entre 0.23 à 0.29, et vaut en moyenne 0.26 (figure 4b).

Selon une mise en œuvre de l'invention, les étapes 1 et 2 décrites ci-dessus peuvent être mises en œuvre au moyen du dispositif ROCK-EVAL® (IFP Energies nouvelles, France), développée par la demanderesse, et décrit notamment dans le brevet EP 2342557 (US 8796035). En effet, le dispositif ROCK-EVAL® comprend au moins :

- un four de pyrolyse en atmosphère non oxydante,

- des moyens d'oxydation des effluents soufrés de pyrolyse
- des moyens de mesure en continu de la quantité de $SO_2$ contenue dans lesdits effluents après oxydation,
- des moyens de transfert des résidus de pyrolyse dans un four d'oxydation,
- un four d'oxydation en atmosphère oxydante,
- des moyens de mesure en continu de la quantité de $SO_2$ contenue dans ladite partie après oxydation.
- En outre ce dispositif peut également comprendre des moyens de mesures des composés hydrocarbonés libérés au cours de la pyrolyse ainsi qu'un moyen de détection du monoxyde de carbone (CO) et du dioxyde de carbone ($CO_2$).

Le procédé peut également être mis en œuvre au moyen d'un unique four de pyrolyse, pouvant fonctionner en atmosphère non oxydante et en atmosphère oxydante, coopérant avec un dispositif de détection et de mesure de la quantité de dioxyde de soufre ($SO_2$).

**Exemples d'application**

Le procédé selon l'invention est appliqué dans un premier exemple d'application à une série de treize échantillons provenant d'une roche connue sous l'appellation « Grey Shale Member », qui est située dans le Toarcien de la « Whitby Mudstone Formation » du bassin sédimentaire de Cleveland, au Royaume-Uni. Le Grey Shale Member est un intervalle d'argiles marines déposées en environnement oxygéné, intercalé de trois niveaux sédimentaires riches en soufre (appelés « sulphur bands » en anglais). Ces trois niveaux ont été sédimentés dans un environnement anoxique (sans oxygène) à euxinique (plus profond, sans oxygène et sous une tranche d'eau riche en sulfures).

Le procédé selon l'invention est également appliqué dans un deuxième exemple d'application à un échantillon provenant d'une roche connue sous l'appellation « Black band », qui est également un intervalle argileux du Toarcien de la « Whitby Mudstone Formation ».

Le procédé selon l'invention est appliqué dans un troisième exemple d'application à une série de huit échantillons provenant d'une roche connue sous l'appellation « Kimmeridge Clay Formation» du bassin sédimentaire de Wessex-Channel dans le Dorset au Royaume-Uni. L'intervalle étudié, d'âge Kimmeridgien-Tithonien, est constitué d'une alternance d'argiles, de marnes et de calcaires.

Le soufre pyritique et le soufre organique présent dans ces échantillons sont déterminés selon le procédé tel que décrit ci-dessus, au moyen du dispositif ROCK-EVAL® (IFP Energies nouvelles, France). Plus précisément, chaque échantillon est placé dans le four de pyrolyse du dispositif ROCK-EVAL® puis on procède à un chauffage de l'échantillon entre 300°C et 650°C, avec une rampe de température de 25°C/min et sous un flux d'azote à 150ml/min. Selon une mise en œuvre de l'invention, les effluents soufrés libérés par chaque échantillon sont entraînés par un flux d'azote vers une chambre de combustion (encore appelée four d'oxydation) du dispositif ROCK-EVAL® où ils sont transformés en $SO_2$ en flux continu, puis le $SO_2$ est entraîné jusqu'au détecteur de $SO_2$ du dispositif ROCK-EVAL® où ils sont quantifiés en continu. A l'issue de la pyrolyse, chaque résidu d'échantillon est transféré depuis le four de pyrolyse vers le four d'oxydation du dispositif ROCK-EVAL® puis on procède à un chauffage de l'échantillon entre 300°C et 850°C ou 1200°C selon la mise en œuvre, avec une rampe de température de 20°C/min et sous un flux d'air à 100ml/min. Les effluents de $SO_2$ libérés par cette oxydation sont entraînés jusqu'au détecteur de $SO_2$ du dispositif ROCK-EVAL® où ils sont quantifiés en continu. La teneur en soufre pyritique et la teneur en soufre organique de chaque échantillon de roche analysé sont déduites par la mise en œuvre du procédé selon l'invention. Puis elles sont comparées à celles obtenues avec la méthode de l'analyse élémentaire du kérogène telle que décrite précédemment (par ICP-AES pour le fer, et par Infrarouge pour le soufre), appelée méthode selon l'art antérieur par la suite.

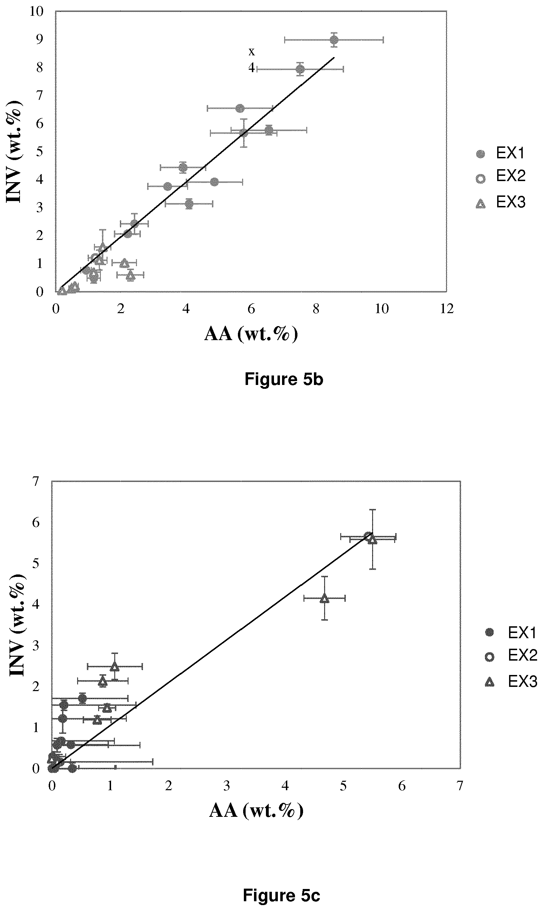

Les figures 5a, 5b, et 5c présentent respectivement l'évolution de la teneur en soufre total, en soufre pyritique et en soufre organique pour chacun des échantillons du premier exemple d'application EX1 (soit les 13 échantillons du « Grey Shale Member »), pour l'échantillon du deuxième exemple d'application EX2 (soit l'échantillon du « Black-Band ») et pour chacun des échantillons du troisième exemple d'application EX3 (soit les 8 échantillons de la « Kimmeridge Clay Formation ») obtenue par le procédé selon l'invention INV, en fonction de la teneur en soufre total obtenue pour ces mêmes échantillons par la méthode selon l'art antérieur AA.

On peut observer sur la figure 5a une très bonne corrélation entre les teneurs en soufre total déterminées à partir du procédé selon l'invention et par la méthode selon l'art antérieur (corrélation avec une pente proche de 1), confirmant la validité de la détermination de la teneur en soufre total d'un échantillon par le procédé selon l'invention.

De même, on peut observer sur la figure 5b une très bonne corrélation entre les teneurs en soufre pyritique déterminées à partir du procédé selon l'invention et par la méthode selon l'art antérieur (corrélation avec une pente proche de 1), confirmant la validité de la détermination de la teneur en soufre pyritique par le procédé selon l'invention.

La figure 5c montre une moins bonne droite de corrélation en ce qui concerne la teneur en soufre organique déterminée par le procédé selon l'invention et par la méthode selon l'art antérieur. Or, dans le procédé selon l'invention, la teneur en soufre organique est entièrement déduite des teneurs en soufre total et pyritique déterminées par le procédé selon l'invention (cf étape 3 ci-dessus), dont les valeurs sont elles valides (cf. discussion relative aux figure 5a et 5b ci-dessus). Ce défaut de corrélation peut être liée au fait que les échantillons sélectionnés ont majoritairement une faible teneur en soufre organique. En effet la teneur en soufre organique des échantillons sélectionnés est majoritairement comprise entre 0wt% et 1wt% (valeurs de l'art antérieur) ou entre 0wt% et 3wt% (valeurs obtenues par le procédé selon l'invention) (figure 5c). Ainsi, il est possible que les teneurs en soufre organique obtenues ici soient de l'ordre de grandeur de 'erreur de mesure de chacune des méthodes. En effet, une répétition des analyses réalisées au moyen du procédé selon l'invention pour différents échantillons montre que l'écart-type sur la mesure est en moyenne de 0.1wt% pour le soufre total, et de 0.2wt% pour le soufre pyritique et organique. Par ailleurs, une répétition des analyses réalisées au moyen du procédé selon l'art antérieur montre que l'écart-type sur la mesure est en moyenne de 0.1wt% pour le soufre total, de 0.5wt% pour le soufre pyritique et de 0.6wt% pour le soufre organique. On constate donc que la méthode selon l'art antérieur semble moins précise que le procédé selon l'invention. Ceci peut s'expliquer par le fait que l'art antérieur combine deux différentes techniques (ICP-AES et Infrarouge) et donc deux sources d'erreur possibles, et par le fait que cet art antérieur se base sur l'hypothèse que tout le fer contenu dans la matière organique est pyritique. Or si le fer contenu dans la matière organique se trouve aussi sous d'autres formes telles que des oxydes ou d'autres sulfures que la pyrite, alors on surestime la teneur en soufre pyritique et l'on sous-estime la teneur en soufre organique. A l'opposé, le procédé selon l'invention ne met en œuvre qu'un seul type de mesure (mesure du $SO_2$ libéré par un échantillon) et se base sur une distinction parfaite entre le soufre pyritique et le soufre organique pendant la phase de pyrolyse. Ainsi, le procédé selon l'invention est moins incertain que l'art antérieur.

Par ailleurs, le procédé selon l'invention est plus rapide puisque la mise en œuvre du procédé selon l'invention sur par exemple les 13 échantillons du Grey Shale Member, exemple d'application décrit ci-dessus, s'est déroulée en environ 15 heures pour les 13 échantillons, alors que la mise en œuvre du procédé selon l'art antérieur sur le même exemple d'application s'est déroulée en environ 7 jours.

## Revendications

1. Procédé pour la quantification du soufre pyritique dans un échantillon de roche sédimentaire, dans lequel on applique au moins les étapes suivantes :

   A. on chauffe ledit échantillon en atmosphère inerte, entre une première température comprise entre 100°C et 320°C et une deuxième température comprise entre 600°C et 700°C, en suivant un premier gradient de température compris entre 1°C/min et 30°C/min ;

   B. on oxyde en continu au moins une partie des effluents issus de ladite chauffe dudit échantillon en atmosphère inerte, on mesure en continu une première quantité de $SO_2$ libérée en fonction du temps de ladite chauffe en atmosphère inerte, et on détermine au moins une teneur en soufre de pyrolyse $S_{Pyrol}$ et une teneur en soufre pyritique de pyrolyse $S_{Pyrol}^{Pyrit}$ à artir de ladite première quantité de $SO_2$ ;

   C. on chauffe en atmosphère oxydante le résidu dudit échantillon issu de ladite chauffe en atmosphère inerte entre une troisième température comprise entre 280°C et 320°C et une quatrième température supérieure ou égale à 800°C, en suivant un second gradient de température compris entre 1°C/min et 30°C/min ;

   D. on mesure en continu une deuxième quantité de $SO_2$ libérée en fonction du temps de ladite chauffe en atmosphère oxydante, on détermine au moins une teneur en soufre d'oxydation $S_{Oxy}$ à partir de ladite deuxième quantité de $SO_2$, et on détermine au moins une teneur en soufre total $S_{Total}$ par la somme de ladite teneur en soufre de pyrolyse $S_{Pyrol}$ et de ladite teneur en soufre d'oxydation $S_{Oxy}$ ;

   **caractérisé en ce qu'**on détermine au moins une teneur en soufre pyritique $S^{Pyrit}$ contenu dans ledit échantillon à partir d'une formule du type :

   $$S^{Pyrit} = p(\alpha, \beta, \gamma).S_{Pyrol}^{Pyrit} \ ,$$

   où $p(\alpha,\beta,\gamma)$ est une fonction de pondération dépendant d'un paramètre $\alpha$ représentant une proportion dudit soufre pyritique de pyrolyse par rapport audit soufre total, d'un paramètre $\beta$ représentant un effet de la matrice minérale sur ladite proportion, d'un paramètre $\gamma$ représentant un effet de la matrice organique sur ladite proportion, les valeurs desdits paramètres étant prédéterminées,
   ladite fonction de pondération $p(\alpha,\beta,\gamma)$ s'écrivant sous la forme :

$$p(\alpha, \beta, \gamma) = \frac{(1 + \beta + \gamma)}{\alpha}$$

et dans lequel :

i) le paramètre $\alpha$ est compris entre 0.40 et 0.46, et vaut préférentiellement 0.43, et
ii) le paramètre $\beta$ est compris entre :

- 0.04 et 0.7, et vaut préférentiellement 0.38 si ledit échantillon de roche est de type argile, ou
- 0.7 et 0.9, et vaut préférentiellement 0.78 si ledit échantillon de roche est de type marne, ou
- entre 0.85 et 0.97, et vaut préférentiellement 0.9 si ledit échantillon de roche est de type calcaire, et

iii) le paramètre $\gamma$ :

- vaut 0 si ledit échantillon de roche contient une matière organique d'origine lacustre et/ou marine, ou
- est compris entre 0.23 et 0.29, et vaut préférentiellement 0.26 si ledit échantillon de roche contient une matière organique d'origine terrestre.

2. Procédé selon la revendication 1, dans lequel ledit échantillon est de type roche réservoir, et dans lequel ladite première température est comprise entre 100°C et 200°C.

3. Procédé selon la revendication 1, dans lequel ledit échantillon est de type roche mère, et dans lequel ladite première température est comprise entre 280°C et 320°C.

4. Procédé selon l'une des revendications précédentes, dans lequel ladite quatrième température est comprise entre 800°C et 900°C, et dans lequel on détermine une teneur en soufre organique $S^{Org}$ selon la formule :

$$S^{Org} = S_{Total} - S^{Pyrit}.$$

5. Procédé selon l'une des revendications 1 à 3, dans lequel ladite quatrième température est supérieure à 1150°C, et préférentiellement inférieure 1250°C, et dans lequel, on détermine en sus une teneur en soufres sulfates $S^{Sulfa}_{Oxy}$ à partir de la dite deuxième quantité de $SO_2$, et on en déduit une teneur en soufre organique selon la formule :

$$S^{Org} = S_{Total} - S^{Pyrit} - S^{Sulfa}_{Oxy}.$$

6. Procédé selon l'une des revendications précédentes, dans lequel on détermine ladite teneur en soufre de pyrolyse et/ou ladite teneur en soufre pyritique de pyrolyse à partir de ladite première quantité de $SO_2$ et d'un coefficient de calibration du soufre de pyrolyse établi sur un échantillon de référence dont on connaît la teneur en soufre.

7. Procédé selon la revendication 6, dans lequel ledit échantillon de référence est du soufre natif.

8. Procédé selon l'une des revendications précédentes, dans lequel on détermine ladite teneur en soufre d'oxydation à partir de ladite deuxième quantité de $SO_2$ et d'un coefficient de calibration du soufre d'oxydation établi sur un échantillon de référence dont on connaît la teneur en soufre.

9. Procédé selon la revendication 8, dans lequel ledit échantillon de référence est un charbon.

10. Procédé selon l'une des revendications précédentes, dans lequel on mesure en outre :

- les quantités de produits hydrocarbonés, de CO et de $CO_2$ contenues dans lesdits effluents issus de ladite chauffe dudit échantillon en atmosphère inerte,
- les quantités de CO et de $CO_2$ contenues dans les effluents issus de ladite chauffe en atmosphère oxydante.

**Patentansprüche**

1. Verfahren zur Quantifizierung von pyritischem Schwefel in einer Sedimentgesteinsprobe, wobei mindestens die folgenden Schritte durchgeführt werden:

   A. Erhitzen der Probe in inerter Atmosphäre zwischen einer ersten Temperatur im Bereich zwischen 100 °C und 320 °C und einer zweiten Temperatur im Bereich zwischen 600 °C und 700 °C, wobei ein erster Temperaturgradient im Bereich zwischen 1 °C/min und 30 °C/min eingehalten wird;

   B. kontinuierliches Oxidieren mindestens eines Teils der Abflüsse, die beim Erhitzen der Probe in inerter Atmosphäre anfallen, kontinuierliches Messen einer ersten $SO_2$-Menge, die in Abhängigkeit von der Dauer des Erhitzens in inerter Atmosphäre freigesetzt wird, und Bestimmen mindestens eines Gehalts an Pyrolyseschwefel $S_{Pyrol}$ und eines Gehalts an pyritischem Pyrolyseschwefel $S_{Pyrol}^{Pyrit}$ aus der ersten $SO_2$-Menge;

   C. Erhitzen in einer oxidierenden Atmosphäre des Rückstands der Probe, der aus dem Erhitzen in inerter Atmosphäre zwischen einer dritten Temperatur im Bereich zwischen 280 °C und 320 °C und einer vierte Temperatur von größer oder gleich 800 °C erhalten wird, wobei ein zweiter Temperaturgradient im Bereich zwischen 1 °C/min und 30 °C/min eingehalten wird;

   D. kontinuierliches Messen einer zweiten $SO_2$-Menge, die in Abhängigkeit von der Dauer des Erhitzens in oxidierender Atmosphäre freigesetzt wird, Bestimmen mindestens eines Gehalts an Oxidationsschwefel $S_{Oxy}$ aus der zweiten $SO_2$-Menge, und Bestimmen mindestens eines Gesamtschwefelgehalts $S_{Total}$ aus der Summe des Gehalts an Pyrolyseschwefel $S_{Pyrol}$ und des Gehalts an Oxidationsschwefel $S_{Oxy}$;

   **dadurch gekennzeichnet, dass** mindestens ein Gehalt an pyritischem Schwefel $S^{Pyrit}$, der in der Probe enthalten ist, bestimmt wird aus einer Formel wie zum Beispiel:

   $$S^{Pyrit} = p(\alpha, \beta, \gamma).S_{Pyrol}^{Pyrit} \quad ,$$

   wobei $p(\alpha, \beta, \gamma)$ eine Gewichtsfunktion in Abhängigkeit von einem Parameter $\alpha$ ist, der für einen Anteil des pyritischen Pyrolyseschwefels bezogen auf den Gesamtschwefel steht, von einem Parameter $\beta$, der für eine Wirkung der mineralischen Matrix auf diesen Anteil steht, von einem Parameter $\gamma$, der für eine Wirkung der organischen Matrix auf diesen Anteil steht, wobei die Werte der Parameter vorbestimmt sind,
   wobei die Gewichtungsfunktion $p(\alpha, \beta, \gamma)$ die Form:

   $$p(\alpha, \beta, \gamma) = \frac{(1+\beta+\gamma)}{\alpha}$$

   aufweist und wobei:

   i) der Parameter $\alpha$ im Bereich zwischen 0,40 und 0,46 liegt und bevorzugt 0,43 ist, und
   ii) der Parameter $\beta$ im Bereich zwischen:

   - 0,04 und 0,7 liegt und bevorzugt 0,38 ist, wenn die Gesteinsprobe ein Ton ist, oder
   - 0,7 und 0,9 liegt und bevorzugt 0,78 ist, wenn die Gesteinsprobe ein Mergel ist, oder
   - zwischen 0,85 und 0,97 liegt und bevorzugt 0,9 ist, wenn die Gesteinsprobe ein Kalkstein ist, und

   iii) der Parameter y:

   - 0 ist, wenn die Gesteinsprobe ein organisches Material limnischen und/oder marinen Ursprungs enthält, oder
   - im Bereich zwischen 0,23 und 0,29 liegt, und bevorzugt 0,26 ist, wenn die Gesteinsprobe ein organisches Material terrestrischen Ursprungs enthält.

2. Verfahren nach Anspruch 1, wobei die Probe ein Lagerstättengestein ist und wobei die erste Temperatur im Bereich zwischen 100 °C und 200 °C liegt.

3. Verfahren nach Anspruch 1, wobei die Probe ein Muttergestein ist und wobei die erste Temperatur im Bereich zwischen 280 °C und 320 °C liegt.

4. Verfahren nach einem der vorhergehenden Ansprüche, wobei die vierte Temperatur im Bereich zwischen 800 °C und 900 °C liegt, und wobei ein Gehalt an organischem Schwefel $S^{Org}$ gemäß der Formel:

$$S^{Org} = S_{Total} - S^{Pyrit},$$

bestimmt wird.

5. Verfahren nach einem der Ansprüche 1 bis 3, wobei die vierte Temperatur größer als 1.150 °C und bevorzugt kleiner als 1.250 °C ist, und wobei außerdem ein Gehalt an Sulfatschwefel $S^{Sulfa}_{Oxy}$ aus der zweiten SO$_2$-Menge bestimmt wird, und daraus ein Gehalt an organischem Schwefel gemäß der Formel:

$$S^{Org} = S_{Total} - S^{Pyrit} - S^{Sulfa}_{Oxy},$$

abgeleitet wird.

6. Verfahren nach einem der vorhergehenden Ansprüche, wobei der Gehalt an Pyrolyseschwefel und/oder der Gehalt an pyritischen Pyrolyseschwefel aus der ersten SO$_2$-Menge und einem Pyrolyseschwefel-Kalibrierungskoeffizienten bestimmt wird, der anhand einer Referenzprobe ermittelt wird, deren Schwefelgehalt bekannt ist.

7. Verfahren nach Anspruch 6, wobei die Referenzprobe nativer Schwefel ist.

8. Verfahren nach einem der vorhergehenden Ansprüche, wobei der Gehalt an Oxidationsschwefel aus der zweiten SO$_2$-Menge und aus einem Oxidationsschwefel-Kalibrierungskoeffizienten bestimmt wird, der aus einer Referenzprobe ermittelt wird, deren Schwefelgehalt bekannt ist.

9. Verfahren nach Anspruch 8, wobei die Referenzprobe eine Kohle ist.

10. Verfahren nach einem der vorhergehenden Ansprüche, wobei ferner gemessen werden:

- die Mengen an Kohlenwasserstoffprodukten, von CO und von CO$_2$, die in den Abflüssen enthalten sind, die beim Erhitzen der Probe in inerter Atmosphäre anfallen,
- die Mengen an CO und an CO$_2$, die in den Abflüssen enthalten sind, die beim Erhitzen in oxidierender Atmosphäre anfallen.

**Claims**

1. Process for quantifying the pyritic sulfur in a sedimentary rock sample, in which at least the following steps are applied:

A. said sample is heated in an inert atmosphere, between a first temperature of between 100°C and 320°C and a second temperature of between 600°C and 700°C, while following a first temperature gradient of between 1°C/min and 30°C/min;
B. at least one portion of the effluents resulting from said heating of said sample in an inert atmosphere is continuously oxidized, a first amount of SO$_2$ released as a function of time of said heating in an inert atmosphere is continuously measured, and at least one content of pyrolysis sulfur $S_{pyrol}$ and one content of pyrolysis pyritic sulfur $S^{Pyrit}_{Pyrol}$ is determined from said first amount of SO$_2$;
C. the residue of said sample resulting from said heating in an inert atmosphere is heated in an oxidizing atmosphere between a third temperature of between 280°C and 320°C and a fourth temperature of greater than or equal to 800°C, while following a second temperature gradient of between 1°C/min and 30°C/min;

D. a second amount of $SO_2$ released as a function of time of said heating in an oxidizing atmosphere is continuously measured, at least one content of oxidation sulfur $S_{oxy}$ is determined from said second amount of $SO_2$, and at least one total sulfur content $S_{Total}$ is determined by the sum of said content of pyrolysis sulfur $S_{pyrol}$ and of said content of oxidation sulfur $S_{oxy}$;

**characterized in that** at least one content of pyritic sulfuric $S^{pyrit}$ contained in said sample is determined on the basis of a formula of the type:

$$S^{Pyrit} = p(\alpha, \beta, \gamma) . S_{Pyrol}^{Pyrit} \quad ,$$

wherein $\rho(\alpha,\beta,\gamma)$ is a weighting function dependent on a parameter $\alpha$ representing a proportion of said pyrolysis pyritic sulfur relative to said total sulfur, on a parameter $\beta$ representing an effect of the mineral matrix on said proportion, and on a parameter $\gamma$ representing an effect of the organic matrix on said proportion, the values of said parameters being predetermined,
said weighting function $\rho(\alpha,\beta,\gamma)$ being written in the form:

$$p(\alpha, \beta, \gamma) = \frac{(1 + \beta + \gamma)}{\alpha}$$

and in which:

i) the parameter $\alpha$ is between 0.40 and 0.46, and is preferentially equal to 0.43, and
ii) the parameter $\beta$ is between:

- 0.04 and 0.7, and is preferentially equal to 0.38 if said rock sample is of clay type, or
- 0.7 and 0.9, and preferentially is equal to 0.78 if said rock sample is of marl type, or
- between 0.85 and 0.97, and is preferentially equal to 0.9 if said rock sample is of limestone type, and

iii) the parameter $\gamma$:

- is equal to 0 if said rock sample contains organic matter of lacustrine and/or marine origin, or
- is between 0.23 and 0.29, and preferentially is equal to 0.26 if said rock sample contains organic matter of terrestrial origin.

2. Process according to Claim 1, in which said sample is of reservoir rock type, and in which said first temperature is between 100°C and 200°C.

3. Process according to Claim 1, in which said sample is of source rock type, and in which said first temperature is between 280°C and 320°C.

4. Process according to one of the preceding claims, in which said fourth temperature is between 800°C and 900°C, and in which an organic sulfur content $S^{Org}$ is determined according to the formula:

$$S^{Org} = S_{Total} - S^{Pyrit}.$$

5. Process according to one of Claims 1 to 3, in which said fourth temperature is greater than 1150°C, and preferentially less than 1250°C, and in which a content of sulfate sulfurs $S_{Oxy}^{Sulfa}$ is in addition determined from said second amount of $SO_2$, and an organic sulfur content is deduced therefrom according to the formula:

$$S^{Org} = S_{Total} - S^{Pyrit} - S_{Oxy}^{Sulfa}.$$

6. Process according to one of the preceding claims, in which said content of pyrolysis sulfur and/or said content of pyrolysis pyritic sulfur are determined from said first amount of $SO_2$ and from a coefficient of calibration of the pyrolysis sulfur established on a reference sample of which the sulfur content is known.

7. Process according to Claim 6, in which said reference sample is native sulfur.

8. Process according to one of the preceding claims, in which said content of oxidation sulfur is determined from said second amount of $SO_2$ and from a coefficient of calibration of the oxidation sulfur established on a reference sample of which the sulfur content is known.

9. Process according to Claim 8, in which said reference sample is coal.

10. Process according to one of the preceding claims, in which the following are also measured:

   - the amounts of hydrocarbon-based products, of CO and $CO_2$ contained in said effluents resulting from said heating of said sample in an inert atmosphere,
   - the amounts of CO and of $CO_2$ contained in the effluents resulting from said heating in an oxidizing atmosphere.

**Figure 1a**

**Figure 1b**

**Figure 2**

**Figure 3a**

**Figure 3b**

**Figure 3c**

**Figure 4a**

**Figure 4b**

**Figure 5a**

**Figure 5b**

**Figure 5c**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2342557 A
- US 8796035 B

**Littérature non-brevet citée dans la description**

- **ACHOLLA, F.V. ; ORR, W.L.** Pyrite removal from kerogen without altering organic matter: The chromous chloride method. *Energy Fuels,* 1993, vol. 7, 406-410
- **BOLIN, T.B.** Direct détermination of pyrite content in Argonne premium coals by the use of sulfur X-ray near edge absorption spectroscopy (S-XANES). *Energy and Fuels,* 2010, vol. 24, 5479-5482
- **CANFIELD, D.E. ; RAISWELL, R. ; WESTRICH, J.T. ; REAVES, C.M. ; BERNER, R.A.** The use of chromium reduction in the analysis of reduced inorganic sulfur in sediments and shales. *Chemical Geology,* 1986, vol. 54, 149-155
- **ORR W.** Kerogen/asphaltene/sulfur relationships in sulfur-rich Monterey oils. *Org. Geochem.,* 1986, vol. 10, 499-516
- **VAIRAVAMURTHY, M.A. ; MALETIC, D. ; WANG, S. ; MANOWITZ, B. ; EGLINTON, T. ; LYONS, T.** Characterization of sulfur-containing functional groups in sedimentary humic substances by X-ray absorption near-edge structure spectroscopy. *Energy and Fuels,* 1997, vol. 11, 546-553
- **VANDENBROUCKE, M. ; LARGEAU, C.** Kerogen origin, evolution and structure. *Organic Geochemistry,* 2007, vol. 38, 719-833